# EUROPÄISCHE PATENTSCHRIFT

(11) **EP 1 035 107 B1**
(45) Veröffentlichungstag und Bekanntmachung des Hinweises auf die Patenterteilung: **14.03.2007**
(21) Anmeldenummer: 00103575.7
(22) Anmeldetag: 19.02.2000
(51) Int. Cl.: C07C 213/08, C07C 215/10

(54) **Verfahren zur Herstellung von N-Alkylpolyhydroxyalkylaminen aus Monoalkylamin und reduzierendem Zucker**
Process for the preparation of N-alkylpolyhydroxyalkylamines from a monoalkylamine and a reducing sugar
Procédé pour la préparation de N-alkylpolyhydroxyalkylamines à partir d'une monoalkylamine et d'un sucre réducteur

(30) Priorität: 06.03.1999 DE 19909907
(43) Veröffentlichungstag der Anmeldung: 13.09.2000
(73) Patentinhaber: Clariant Produkte (Deutschland) GmbH, 65929 Frankfurt am Main (DE)
(72) Erfinder: Gallas, Andreas, 84567 Burgkirchen (DE); Hanauer, Johann Franz, Dr., 84579 Unterneukirchen (DE); Seitz, Hubert, Dr., 4153 Reinach/BL (CH); Weinelt, Frank, Dr., 84508 Burgkirchen (DE)

(56) Entgegenhaltungen:
- WO-A-93/03004
- US-A- 2 016 962
- US-A- 5 334 764

## Beschreibung

Die Erfindung betrifft ein Verfahren zur Herstellung von N-Alkylpolyhydroxyalkylaminen aus Monoalkylamin und reduzierendem Zucker, wobei man zunächst das Alkylamin und den reduzierenden Zucker in Lösung zusammenmischt, die erhaltene Lösung mit Wasserstoff in Gegenwart eines Hydrierkatalystors hydriert und das gebildete N-Alkylpolyhydroxyalkylamin durch Abtrennen vom Hydrierkatalysator gewinnt.

Ein solches Verfahren ist zum Beispiel aus US-A-2 016 962, WO-A-92/06073, WO-A-92/08687, WO-A-93/03004 und EP-A-0 536 939 bekannt, wobei in den drei WO-Druckschriften eine ausführliche Beschreibung über das Inkontaktbringen von Monoalkylamin und Zucker gegeben wird. Es erfolgt im wesentlichen in der Weise, daß der Zucker und überschüssiges Alkylamin bei einer Temperatur von etwa 10 bis 60°C und Atmosphärendruck zusammengebracht werden. So werden in Beispiel I von WO 93/03004 eine wäßrige Methylaminlösung, Glucose und Ethanol bei Raumtemperatur zusammengemischt und die erhaltene Lösung wird über Nacht stehengelassen. In den Beispielen IX, XI und XII wird eine wäßrige Glucoselösung bei einer Temperatur von 10 bis 20°C und wiederum Atmosphärendruck langsam zu einer wäßrigen Methylaminlösung gegeben, worauf die Lösung in Beispiel XI noch etwa 30 Minuten lang und im Beispiel XII etwa 2 Stunden lang gerührt wird.

Wie aus dem Stand der Technik hervorgeht, insbesondere aus WO-A 93/03004, kommt der Bereitung der Lösung oder Mischung aus dem Zucker und dem Alkylamin, die dann hydriert wird, eine bedeutende Rolle zu bezüglich Ausbeute an N-Alkylpolyhydroxyalkylamin und seinem Gehalt an färbenden Nebenprodukten und anderen Verunreinigungen.

Es wurde überraschenderweise gefunden, daß man eine hohe Ausbeute an N-Alkylpolyhydroxyalkylamin mit hoher Farbqualität und Reinheit erhält, wenn man die zu hydrierende Lösung in der Weise bereitet, daß man das N-Alkylamin und den Zucker jeweils als Lösung unter Anwendung von Druck und turbulenter Strömung höchstens 5 Minuten lang mischt. Es ist ferner vorteilhaft, wenn man die Lösung unmittelbar nach Ablauf der genannten Mischzeit den Hydrierbedingungen aussetzt.

Das erfindungsgemäße Verfahren ist demnach dadurch gekennzeichnet, daß das Zusammenmischen von Monoalkylamin und Zucker in der Weise durchgeführt wird, daß man das Monoalkylamin und den reduzierenden Zucker jeweils in Form einer wäßrigen, alkoholischen oder wäßrig/alkoholischen Lösung gleichzeitig in eine Mischeinheit eindrückt und die Lösung in der Mischeinheit bei einer Temperatur von 25 bis 60°C, vorzugsweise 40 bis 50°C, und einem Druck von 50 bis 90 bar, vorzugsweise 60 bis 80 bar, 3 Sekunden bis 5 Minuten lang, vorzugsweise 10 Sekunden bis 1 Minute lang, unter Turbulenz hält.

Das Alkylamin wird vorzugsweise als 10 bis 50 gew.-%ige Lösung und der Zucker als 40 bis 75 gew.-%ige Lösung in Wasser, Alkohol oder in einer Mischung aus Wasser und Alkohol eingesetzt, wobei Wasser allein als Lösungsmittel bevorzugt ist. Die Alkylaminlösung wird vorzugsweise bei einer Temperatur von 10 bis 30°C und die Zuckerlösung bei einer Temperatur von 40 bis 60°C der Mischstrecke zugeführt.

Während des Mischens der beiden Lösungen wird Wärme frei (exotherme Reaktion). Die angegebene Temperatur der Lösungen, die in die Mischstrecke eingeführt werden, ist abgestimmt auf die beim Mischen einzuhaltende Temperatur von 25 bis 60°C, vorzugsweise 40 bis 50°C, unter Berücksichtigung der exothermen Reaktion, so daß die genannte Mischtemperatur ohne weitere Maßnahmen (wie Kühlen oder Erwärmen) gewährleistet ist. Im Falle von Alkohol als Lösungsmittel sind die C₁-C₄-Alkanole, wie Methanol, Ethanol und Isopropanol und die niedermolekularen Glykole wie Monoethylenglykol, Diethylenglykol und Propylenglykol oder Mischungen aus diesen Alkoholen und Glykolen bevorzugt.

Das Mischen der Alkylaminlösung und der Zuckerlösung erfolgt erfindungsgemäß in einer turbulenten Strömung unter Einhaltung der angegebenen Temperatur- und Druckwerte und der angegebenen Zeit (Verweilzeit), während der unter Turbulenz gemischt werden soll. Als Mischstrecke oder Mischeinheit wird vorzugsweise ein Rohr verwendet, das dem einzuhaltenden Druck standhält. Turbulenz kann zum Beispiel dadurch erreicht werden, daß man im druckfesten Rohr die bekannten turbulenzerzeugenden Einbauten anbringt. Solche Rohre werden bekanntlich als Statikmischer bezeichnet. Zur Einhaltung der erfindungsgemäßen Verweilzeit von 6 Sekunden bis 5 Minuten, vorzugsweise 10 Sekunden bis 1 Minute, ist klarerweise der Durchmesser des Rohres der eingesetzten Lösungsmenge anzupassen. Die Alkylaminlösung und die Zuckerlösung werden (vorzugsweise getrennt voneinander) gleichzeitig in die Mischeinheit eingeführt, wobei klarerweise ein etwas höherer Druck im Vergleich zum Druck in der Mischeinheit anzuwenden ist.

Die nach dem Mischen in der Mischstrecke resultierende Lösung wird vorzugsweise sofort der Hydrierung unterworfen. Um eine unmittelbar anschließende Hydrierung zu erreichen, ist es bevorzugt, die Lösung nach der angegebenen Verweilzeit in der Mischstrecke direkt in einen Rührautoklaven einzudrücken, in dem der Hydrierkatalysator bereits vorgelegt ist und in dem auch schon die Hydriertemperatur und der zur Hydrierung erforderliche Wasserstoffdruck eingestellt sind. Dieser Wasserstoffdruck liegt bei 25 bis 100 bar, vorzugsweise 40 bis 80 bar, und die Hydriertemperatur bei 40 bis 85°C, vorzugsweise 50 bis 80°C. Diese Hydrierbedingungen werden erfindungsgemäß solange aufrechterhalten, bis praktisch kein Wasserstoff mehr aufgenommen wird (die Hydrierzeit beträgt im allgemeinen 30 Minuten bis 5 Stunden).

Nach einer bevorzugten Ausführungsform des erfindungsgemäßen Verfahrens wird die hydrierte Lösung zunächst thermisch stabilisiert und erst dann das gebildete N-Alkylpolyhydroxyalkylamin abgetrennt. Die thermische Stabilisierung wird vorzugsweise im Anschluß an die Hydrierung im Rührautoklaven selbst durchgeführt, wobei die hydrierte Lösung (mit der genannten Temperatur von 40 bis 100°C, vorzugsweise 25 bis 85°C, und unter einem Wasserstoffdruck von 50 bis 80 bar, vorzugsweise 40 bis 80 bar) auf 95 bis 100°C, vorzugsweise 95 bis 105°C, erhitzt wird. Die Steigerung der Temperatur auf diese höheren Werte unter Beibehaltung des anwesenden Wasserstoffs erfolgt vorzugsweise kontinuierlich und langsam, das heißt, in einer Zeit von etwa 30 bis 150 Minuten, vorzugsweise 40 bis 110 Minuten (es versteht sich von selbst, daß mit der Temperatursteigerung auch der Wasserstoffdruck im Autoklaven mehr oder weniger ansteigt). Sobald die Lösung die höheren Temperaturwerte erreicht hat, wird sie durch Abkühlen auf eine Temperatur von 60 bis 90°C gebracht, vorzugsweise 70 bis 80°C, was vorzugsweise schnell geschehen sollte (15 bis 30 Minuten). Während der Stabilisierungsphase, bestehend aus Temperaturerhöhung und Temperatursenkung, wird von der hydrierten Lösung praktisch kein Wasserstoff aufgenommen, und es wird dem System, wie oben bereits erwähnt, Wasserstoff nicht nennenswert zugeführt noch entnommen.

Nach der Hydrierung und der gegebenenfalls durchgeführten thermischen Stabilisierung wird das N-Alkylpolyhydroxyalkylamin aus dem Reaktionsprodukt gewonnen. Dies erfolgt erfindungsgemäß bevorzugt in der Weise, daß man zunächst das Reaktionsprodukt im Rührautoklaven, in dem die Hydrierung und gegebenenfalls thermische Stabilisierung durchgeführt worden ist, unter Stillegung der Rührung einer Sedimentationsphase aussetzt. Während dieser Phase wird eine Temperatur von 60 bis 90°C, vorzugsweise 70 bis 80°C, und ein Wasserstoffdruck von 70 bis 95 bar, vorzugsweise 80 bis 90 bar, eingestellt (sofern diese Temperatur und dieser Druck nicht ohnehin schon vorliegen) und aufrechterhalten. In der Sedimentationsphase (die im allgemeinen 10 bis 40 Minuten dauert) setzt sich der Hydrierkatalystor am Boden des Rührautoklaven ab, und man erhält eine im wesentliche klare Lösung, die das N-Alkylpolyhydroxyalkylamin enthält. Die klare Lösung wird abdekantiert (abgedrückt), vorzugsweise mit Hilfe eines in die Lösung eingeführten Tauchrohres. Während des Abdekantierens wird im wesentlichen die gleiche Temperatur und der gleiche Wasserstoffdruck wie bei der Sedimentation aufrechterhalten. Die abdekantierte, im wesentlichen klare Lösung kann gegebenenfalls noch filtriert werden. Ein Abdestillieren des Lösungsmittels wird man dann vornehmen, wenn man das N-Alkylpolyhydroxyalkylamin lösungsmittelfrei haben möchte.

Beim erfindungsgemäßen Verfahren werden das Monoalkylamin und die Polyolverbindung im Molverhältnis von etwa 1 bis 2 zu 1 eingesetzt, vorzugsweise 1 bis 1,6 zu 1 (das stöchiometrische Molverhältnis ist 1 zu 1). Es wurde gefunden, daß es vorteilhaft ist, wenn man Monoalkylamin auch im Hydrierautoklaven vorlegt, in welchem die unter Turbulenz gemischte Lösung zur Hydrierung eingedrückt wird. Die Menge an vorgelegtem Alkylamin kann in weiten Grenzen variieren und liegt im allgemeinen bei 0,05 bis 3 mol, vorzugsweise 0,1 bis 1 mol Alkylamin pro mol Polyolverbindung. Zur Vermeidung eines zu großen Gesamtüberschusses an Alkylamin ist es bevorzugt, die ganze oder zumindest einen größeren Teil der vorzulegenden Alkylaminmenge von der überstöchiometrischen Menge im genannten Verhältnis 1 bis 2 zu 1 (vorzugsweise 1 bis 1,6 zu 1) abzuzweigen. Von dieser überstöchiometrischen Menge an Alkylamin wird man also etwa 30 bis 100 Gew.-%, vorzugsweise 50 bis 90 Gew.-%, im Hydriersystem vorlegen.

Beim erfindungsgemäßen Verfahren werden als Hydrierkatalysatoren vorzugsweise Nickelkatalystoren eingesetzt, wobei Raney-Nickel besonders bevorzugt ist. Die Menge an Hydrierkatalysator kann in weiten Grenzen variieren und liegt im allgemeinen bei 2 bis 20 Gew.-%, vorzugsweise 5 bis 15 Gew.-%, bezogen auf die Menge an eingesetzter Polyolverbindung.

Zu den Ausgangsverbindungen, Monoalkylamin und reduzierender Zucker, sei noch folgendes gesagt:

Das Monoalkylamin ist vorzugsweise ein solches der Formel RHN₂, worin R eine vorzugsweise gerade und gesättigte Alkylgruppe mit 1 bis 18 C-Atomen ist, vorzugsweise mit 1 bis 4 C-Atomen, oder eine Hydroxyalkylgruppe, vorzugsweise C₁-C₄-Hydroxyalkyl. Beispiele sind Methylamin, Ethylamin, Propylamin, Isopropylamin, 2-Hydroxyethylamin, 2-Hydroxypropylamin und dergleichen. Methylamin oder Ethylamin ist besonders bevorzugt. Als Monoalkylamine kommen weiterhin auch C₁-C₄-Dialkylamino-C₂-C₆-alkylamine, beispielsweise Dimethylaminopropylamin oder Alkoxyalkylamine der Formel RO-(CH₂)ₙ-NH₂, worin R C₁-C₄-Alkyl und n eine Zahl von 2 bis 4 bedeutet, in Frage.

Als Polyhydroxyalkylverbindung oder reduzierende Zuckerverbindung werden bevorzugt eingesetzt Monosaccharide, vorzugsweise Pentosen und Hexosen, und Oligosaccharide, vorzugsweise Disaccharide und Trisaccharide. Beispiele für Monosaccharide sind Fructose, Glucose, Galaktose, Mannose, Sorbose und Talose als Hexosen und Arabinose, Ribose und Xylose als Pentosen. Beispiele für Oligosaccharide (Polysaccharide) sind Lactose, Maltose, Maltotriose und dergleichen. Von den Oligosacchariden sind die Disaccharide bevorzugt. Besonders bevorzugte Polyole sind die Hexosen, insbesondere die Glucose.

Die mit dem erfindungsgemäßen Verfahren hergestellten N-Alkylpolyhydroxyalkylamine entsprechen also der Formel R-NH-Z, worin R die genannte Bedeutung hat und Z ein Rest der genannten Polyhydroxyalkylverbindungen ist. Z ist demnach vorzugsweise ein Rest der Formel -CH₂-[-CH(OH)]ₙ-CH₂OH, worin n eine ganze Zahl von 3 bis 5 ist, vorzugsweise 3 oder 4 und besonders bevorzugt 4 ist. Es werden also bevorzugt N-C₁ bis C₃-Glucamine hergestellt, vorzugsweise von Fructose, Glucose, Galaktose, Mannose, Sorbose oder Talose oder von deren Gemischen und besonders bevorzugt die entsprechenden Glucamine wie N-Methylglucamin und
N-Ethylglucamin.

Mit dem erfindungsgemäßen Verfahren wird ein lineares N-Alkylpolyhydroxyalkylamin erhalten, das sehr rein und im kristallinen Zustand farblos ist. Es wird ferner in einer Ausbeute von bis zu 98 Gew.-% erhalten. Dies ist eine sehr hohe Ausbeute angesichts der hohen Reinheit und Farbqualität des Produktes. Das erhaltene Alkylpolyhydroxyalkylamin weist ferner einen nur sehr geringen Gehalt an Hydrierkatalysator (Nickel) auf. Es zeigt auch eine hohe thermische Stabilität, das heißt es behält das farblose Aussehen im wesentlichen bei, auch wenn man das Produkt längere Zeit auf relativ hohe Temperaturen erhitzt. Mit dem erfindungsgemäßen Verfahren wird ferner erreicht, daß die Aktivität des Hydrierkatalystors weitgehend erhalten bleibt, daß also der Katalysator für mehrere Ansätze bei gleicher Ausbeute und Produktqualität zur Verfügung steht. Sobald der gebrauchte Katalysator die gewünschte Aktivität nicht mehr aufweist, ist es möglich, seine ursprüngliche Aktivität wieder herzustellen durch Zusatz einer relativ geringen Menge an frischem Katalysator. Das erfindungsgemäße Verfahren gewährleistet also eine hohe Produktausbeute und Produktqualität und zusätzlich dazu einen nur geringen Verlust an Hydrierkatalysator und Katalysatoraktivität.

Die Erfindung wird nun an Beispielen noch näher erläutert.

### Beispiel 1

146 mol Monomethylamin (MMA) in Wasser (20,6 kg MMA-Lösung) wurden vorgelegt und mit 7,5 kg Raney-Nickel-Wassergemisch als 70 %iger Slurry versetzt. Das Gemisch wurde mit 80 bar Wasserstoff beaufschlagt und auf 50°C erwärmt. Unter Rühren wurden in die MMA-Lösung eine Mischung aus 290 mol Glucose als 70 %iger wäßriger Sirup und 290 mol MMA in Wasser (40,9 kg MMA-Lösung) über einen Statikmischer in den Hydrierreaktor eindosiert. Die mittlere Verweilzeit in der Mischeinheit betrug 5 Sekunden. Die Mischeinheit wurde auf 45°C temperiert. Während der Dosierung wurde der Wasserstoffdruck zwischen 70 und 95 bar und die Temperatur zwischen 48 und 52°C gehalten. Nach beendeter Zugabe wurde bei dem genannten Druck und der genannten Temperatur gehalten bis keine Wasserstoffaufnahme erkennbar war, was etwa 90min beansprucht. Nach Abschluß der Hydrierung erfolgte die Aufheizung des Reaktionsgemisches auf 100°C innerhalb von 60 min. Nach Erreichen der Temperatur wurde innerhalb von 20 min auf 80°C abgekühlt und der Rührer abgestellt. Nach einer Sedimentationszeit von 30 min bei 80°C und etwa 85 bar wurde die überstehende Lösung vom Katalysator abdekantiert und abfiltriert.

Nach Entfernung des überschüssigen Amins und des Lösungsmittels wurden weiße Kristalle erhalten, die ohne Verfärbung bei 130°C schmelzen.

### Beispiel 2

146 mol N,N-Dimethylaminopropylamin (DMAPA) (30,9 kg DMAPA-Lösung) wurden vorgelegt und mit 5,6 kg Raney-Nickel-Wassergemisch als 70 %iger Slurry versetzt. Das Gemisch wurde mit 70 bar Wasserstoff beaufschlagt und auf 65°C erwärmt. Unter Rühren wurden in die DMAPA-Lösung eine Mischung aus 290 mol Glucose als 70 %iger wäßriger Sirup und 290 mol DMAPA in Wasser (61,4 kg DMAPA-Lösung) über einen Statikmischer in den Hydrierreaktor eindosiert. Die mittlere Verweilzeit in der Mischeinheit betrug 60 Sekunden. Die Mischeinheit wurde auf 45°C temperiert. Während der Dosierung wurde der Wasserstoffdruck im Reaktionsgefäß zwischen 50 und 60 bar und die Temperatur zwischen 58 und 62°C gehalten. Nach beendeter Zugabe wurde bei dem genannten Druck und der genannten Temperatur gehalten bis keine Wasserstoffaufnahme erkennbar war, was etwa 180 min beansprucht.

Nach Abschluß der Hydrierung erfolgte die Aufheizung des Reaktionsgemisches auf 105°C innerhalb von 80 min. Nach Erreichen der Temperatur wurde innerhalb von 35 min auf 70°C abgekühlt und der Rührer abgestellt. Nach einer Sedimentationszeit von 20 min bei 70°C und etwa 90 bar wurde die überstehende Lösung vom Katalysator abdekantiert und abfiltriert.

### Beispiel 3

58 mol Methoxypropylamin (MOPA) in Wasser (25,8 kg MOPA-Lösung) wurden vorgelegt und mit 11,25 kg Raney-Nickel-Wassergemisch als 70 %iger Slurry versetzt. Das Gemisch wurde mit 40 bar Wasserstoff beaufschlagt und auf 70°C erwärmt. Unter Rühren wurden in die MOPA-Lösung eine Mischung aus 290 mol Glucose als 70 %iger wäßiger Sirup und 290 mol MOPA in Wasser (129 kg MOPA-Lösung) über einen Statikmischer in den Hydrierreaktor eindosiert. Die mittlere Verweilzeit in der Mischeinheit betrug 15 Sekunden. Die Mischeinheit wurde auf 40°C temperiert. Während der Dosierung wurde der Wasserstoffdruck im Reaktionsgefäß zwischen 80 und 90 bar und die Temperatur zwischen 65 und 68°C gehalten. Nach beendeter Zugabe wurde bei dem genannten Druck und der genannten Temperatur gehalten bis keine Wasserstoffaufnahme erkennbar war, was etwa 120 min beansprucht.

Nach Abschluß der Hydrierung erfolgte die Aufheizung des Reaktionsgemisches auf 95°C innerhalb von 50 min. Nach Erreichen der Temperatur wurde innerhalb von 30 min auf 80°C abgekühlt und der Rührer abgestellt. Nach einer Sedimentationszeit von 10 min bei 80°C und etwa 85 bar wurde die überstehende Lösung vom Katalysator abdekantiert und abfiltriert.

## Patentansprüche

1. Verfahren zur Herstellung von N-Alkylpolyhydroxyalkylaminen aus Monoalkylamin und reduzierendem Zucker, wobei man zunächst das Alkylamin und den reduzierenden Zucker in Lösung zusammenmischt, die erhaltene Lösung mit Wasserstoff in Gegenwart eines Hydrierkatalysators hydriert und das gebildete N-Alkylpolyhydroxyalkylamin durch Abtrennen vom Hydrierkatalysator gewinnt, **dadurch gekennzeichnet, daß** das Zusammenmischen in der Weise durchgeführt wird, daß man das Monoalkylamin und den reduzierenden Zucker jeweils in Form einer wäßrigen, alkoholischen oder wäßrig/alkoholischen Lösung gleichzeitig in eine Mischeinheit eindrückt und die Lösung in der Mischeinheit bei einer Temperatur von 25 bis 60°C und einem Druck von 50 bis 90 bar 6 Sekunden bis 5 Minuten lang unter Turbulenz hält.

2. Verfahren nach Anspruch 1, **dadurch gekennzeichnet, daß** man das Alkylamin in Form einer 10 bis 50 gew.-%igen Lösung und den reduzierenden Zucker in Form einer 40 bis 75 gew.-%igen Lösung in die Mischeinheit eindrückt.

3. Verfahren nach Anspruch 1, **dadurch gekennzeichnet, daß** man das Alkylamin in Form einer 10 bis 50 gew.-%igen wäßrigen Lösung mit einer Temperatur von 10 bis 30°C und den reduzierenden Zucker in Form einer 40 bis 75 gew.-%igen wäßrigen Lösung mit einer Temperatur von 40 bis 60°C in die Mischeinheit eindrückt.

4. Verfahren nach einem oder mehreren der Ansprüche 1 bis 3, **dadurch gekennzeichnet, daß** man die Lösung in der Mischeinheit bei einer Temperatur von 40 bis 50°C und einem Druck von 60 bis 80 bar 6 Sekunden bis 5 Minuten lang unter Turbulenz hält.

5. Verfahren nach einem oder mehreren der Ansprüche 1 bis 4, **dadurch gekennzeichnet, daß** man die Lösung in der Mischeinheit 10 Sekunden bis 1 Minute lang unter Turbulenz hält.

6. Verfahren nach einem oder mehreren der Ansprüche 1 bis 5, **dadurch gekennzeichnet, daß** man das Mischen unter Turbulenz in einem druckfesten Rohr als Mischeinheit durchführt.

7. Verfahren nach einem oder mehreren der Ansprüche 1 bis 6, **dadurch gekennzeichnet, daß** man die nach Ablauf der genannten Mischzeit erhaltene Lösung zur Hydrierung in einen Rührautoklaven eindrückt und hydriert.

8. Verfahren nach Anspruch 7, **dadurch gekennzeichnet, daß** die Hydrierung in der Weise durchgeführt wird, daß man die Lösung in einen Rührautoklaven eindrückt, in welchem Raney-Nickel als Hydrierkatalysator bei einer Temperatur von 40 bis 85°C und einem Wasserstoffdruck von 25 bis 100 bar vorliegt, und die eingedrückte Lösung bei dieser Temperatur und diesem Wasserstoffdruck hydriert, bis im wesentlichen kein Wasserstoff mehr aufgenommen wird.

9. Verfahren nach Anspruch 7 oder 8, **dadurch gekennzeichnet, daß** die hydrierte Lösung einer thermischen Stabilisierung unterworfen wird, indem die Lösung unter Beibehaltung des anwesenden Wasserstoffs in einer Zeit von 30 bis 150 Minuten durch kontinuierliche Temperatursteigerung auf 95 bis 110°C gebracht und nach Erreichen dieser Temperatur auf 60 bis 90°C abgekühlt wird.

10. Verfahren nach einem oder mehreren der Ansprüche 1 bis 9, **dadurch gekennzeichnet, daß** die Trennung des hydrierten und thermisch stabilisierten Produktes, das im wesentlichen aus N-Alkylpolyhydroxyalkylamin und Hydrierkatalysator besteht, in der Weise durchgeführt wird, daß man den Hydrierkatalysator aus dem Produkt bei einer Temperatur von 60 bis 90°C und einem Wasserstoffdruck von 70 bis 95 bar absedimentieren läßt und das darüberstehende N-Alkylpolyhydroxyalkylamin unter Beibehaltung dieser Temperatur und dieses Wasserstoffdruckes abdekantiert.

11. Verfahren nach einem oder mehreren der Ansprüche 1 bis 10, **dadurch gekennzeichnet, daß** man das Monoalkylamin und den reduzierenden Zucker im Molverhältnis von 1 bis 2 : 1 einsetzt.

12. Verfahren nach Anspruch 1, **dadurch gekennzeichnet, daß** man
a) das Monoalkylamin in Form einer 10 bis 50 gew.-%igen wäßrigen, alkoholischen oder wäßrig/alkoholischen Lösung und den reduzierenden Zucker in Form einer 40 bis 95 gew.-%igen wäßrigen, alkoholischen oder wäßrig/alkoholischen Lösung und im Molverhältnis von 1 bis 1,6 mol Alkylamin pro mol Zucker gleichzeitig in eine druckfestes Rohr als Mischeinheit eindrückt und die Lösung im Rohr bei einer Temperatur von 25 bis 60°C und einem Druck von 50 bis 90 bar 6 Sekunden bis 5 Minuten lang unter Turbulenz hält,
b) die im Schritt a) erhaltene Lösung nach Ablauf der genannten Mischzeit in einen Rührautoklaven eindrückt, in welchem 0,1 bis 1 mol Monoalkylamin pro mol eingesetzten Zucker und Raney-Nickel als Hydrierkatalysator bei einer Temperatur von 40 bis 85°C und einem Wasserstoffdruck von 50 bis 80 bar vorliegen, und die eingedrückte Lösung bei dieser Temperatur und diesem Wasserstoffdruck hydriert, bis im wesentlichen kein Wasserstoff mehr aufgenommen wird, und die hydrierte Lösung einer thermischen Stabilisierung unterwirft, indem die Lösung unter Beibehaltung des anwesenden Wasserstoffs in einer Zeit von 60 bis 150 Minuten durch kontinuierliche Temperatursteigerung auf 95 bis 100°C gebracht und nach Erreichen dieser Temperatur auf 60 bis 90°C abgekühlt wird, und daß man
c) die Trennung des hydrierten und thermisch stabilisierten Produktes, das im wesentlichen aus N-Alkylpolyhydroxyalkylamin und Hydrierkatalysator besteht, in der Weise durchführt, daß man den Hydrierkatalysator aus dem Produkt bei einer Temperatur von 60 bis 90°C und einem Wasserstoffdruck von 70 bis 95 bar absedimentieren läßt und das darüberstehende N-Alkylpolyhydroxyalkylamin unter Beibehaltung dieser Temperatur und dieses Wasserstoffdruckes abdekantiert.

13. Verfahren nach Anspruch 12, **dadurch gekennzeichnet, daß** man im Schritt a) das Monoalkylamin in Form einer 10 bis 50 gew.-%igen wäßrigen Lösung mit einer Temperatur von 10 bis 30°C und den reduzierenden Zucker in Form einer 40 bis 75 gew.-%igen wäßrigen Lösung mit einer Temperatur von 40 bis 60°C und im Molverhältnis von 1 bis 1,6 mol Alkylamin pro mol Zucker gleichzeitig in das Rohr eindrückt und die Lösung im Rohr bei einer Temperatur von 40 bis 50°C und einem Druck von 60 bis 80 bar 10 Sekunden bis 1 Minute lang unter Turbulenz hält.

## Claims

1. A process for the preparation of N-alkylpolyhydroxyalkylamines from monoalkylamine and reducing sugar, in which the alkylamine and the reducing sugar are first mixed together in solution, the solution obtained is hydrogenated with hydrogen in the presence of a hydrogenation catalyst and the N-alkylpolyhydroxyalkylamine formed is obtained by removal of the hydrogenation catalyst, which comprises carrying out the mixing together by injecting the monoalkylamine and the reducing sugar, in each case in the form of an aqueous, alcoholic or aqueous/alcoholic solution, simultaneously into a mixing unit and keeping the solution in the mixing unit under turbulence at a temperature of 25 to 60°C and a pressure of 50 to 90 bar for 6 seconds to 5 minutes.

2. The process as claimed in claim 1, wherein the alkylamine is injected into the mixing unit in the form of a 10 to 50% strength by weight solution and the reducing sugar in the form of a 40 to 75% strength by weight solution.

3. The process as claimed in claim 1, wherein the alkylamine is injected into the mixing unit in the form of a 10 to 50% strength by weight aqueous solution having a temperature of 10 to 30°C and the reducing sugar in the form of a 40 to 75% strength by weight aqueous solution having a temperature of 40 to 60°C.

4. The process as claimed in one or more of claims 1 to 3, wherein the solution is kept under turbulence in the mixing unit at a temperature of 40 to 50°C and a pressure of 60 to 80 bar for 6 seconds to 5 minutes.

5. The process as claimed in one or more of claims 1 to 4, wherein the solution is kept under turbulence in the mixing unit for 10 seconds to 1 minute.

6. The process as claimed in one or more of claims 1 to 5, wherein the mixing is carried out under turbulence in a pressure-resistant tube as a mixing unit.

7. The process as claimed in one or more of claims 1 to 6, wherein the solution obtained after expiry of the mixing time mentioned is injected into a stirring autoclave for the hydrogenation and hydrogenated.

8. The process as claimed in claim 7, wherein the hydrogenation is carried out by injecting the solution into a stirring autoclave in which Raney nickel is present as a hydrogenation catalyst at a temperature of 40 to 85°C and a hydrogen pressure of 25 to 100 bar, and hydrogenating the injected solution at this temperature and this hydrogen pressure until hydrogen is essentially no longer absorbed.

9. The process as claimed in claim 7 or 8, wherein the hydrogenated solution is subjected to a thermal stabilization by bringing the solution to 95 to 110°C while retaining the hydrogen present in a time from 30 to 150 minutes by continuous temperature increase and, after reaching this temperature, cooling to 60 to 90°C.

10. The process as claimed in one or more of claims 1 to 9, wherein the separation of the hydrogenated and thermally stabilized product, which essentially consists of N-alkylpolyhydroxyalkylamine and hydrogenation catalyst, is carried out by allowing the hydrogenation catalyst to sediment off from the product at a temperature of 60 to 90°C and a hydrogen pressure of 70 to 95 bar and decanting off the supernatant N-alkylpolyhydroxyalkylamine while retaining this temperature and this hydrogen pressure.

11. The process as claimed in one or more of claims 1 to 10, wherein the monoalkylamine and the reducing sugar are employed in the molar ratio of 1 to 2:1.

12. The process as claimed in claim 1, wherein
a) the monoalkylamine in the form of a 10 to 50% strength by weight aqueous, alcoholic or aqueous/alcoholic solution and the reducing sugar in the form of a 40 to 95% strength by weight aqueous, alcoholic or aqueous/alcoholic solution and in the molar ratio of 1 to 1.6 mol of alkylamine per mole of sugar are injected simultaneously into a pressure-resistant tube as a mixing unit and the solution in the tube is kept under turbulence at a temperature of 25 to 60°C and a pressure of 50 to 90 bar for 6 seconds to 5 minutes,
b) the solution obtained in step a) is injected, after expiry of the mixing time mentioned, into a stirring autoclave in which 0.1 to 1 mol of monoalkylamine per mole of sugar employed and Raney nickel as a hydrogenation catalyst are present at a temperature of 40 to 85°C and a hydrogen pressure of 50 to 80 bar, and the injected solution is hydrogenated at this temperature and this hydrogen pressure until hydrogen is essentially no longer absorbed, and the hydrogenated solution is subjected to a thermal stabilization by bringing the solution to 95 to 100°C while retaining the hydrogen present in a time of 60 to 150 minutes by continuous temperature increase and, after reaching this temperature, cooling to 60 to 90°C, and wherein
c) the separation of the hydrogenated and thermally stabilized product, which essentially consists of N-alkylpolyhydroxyalkylamine and hydrogenation catalyst is carried out by allowing the hydrogenation catalyst to sediment off from the product at a temperature of 60 to 90°C and a hydrogen pressure of 70 to 95 bar and decanting off the supernatant N-alkylpolyhydroxyalkylamine while retaining this temperature and this hydrogen pressure.

13. The process as claimed in claim 12, wherein, in step a), the monoalkylamine in the form of a 10 to 50% strength by weight aqueous solution having a temperature of 10 to 30°C and the reducing sugar in the form of a 40 to 75% strength by weight aqueous solution having a temperature of 40 to 60°C and in the molar ratio of 1 to 1.6 mol of alkylamine per mole of sugar are injected simultaneously into the tube and the solution in the tube is kept under turbulence at a temperature of 40 to 50°C and a pressure of 60 to 80 bar for 10 seconds to 1 minute

## Revendications

1. Procédé de préparation de N-alkylpolyhydroxyalkylamines à partir d'une monoalkylamine et d'un sucre réducteur, dans lequel on commence par mélanger l'alkylamine et le sucre réducteur en solution, on hydrogène la solution obtenue avec de l'hydrogène en présence d'un catalyseur d'hydrogénation et on récupère la N-alkylpolyhydroxyalkylamine formée par séparation du catalyseur d'hydrogénation, **caractérisé en ce que** le mélange est réalisé de telle manière que l'on injecte simultanément la monoalkylamine et le sucre réducteur, chacun sous forme de solution aqueuse, alcoolique ou aqueuse/alcoolique, dans une unité de mélange et que l'on maintient la solution dans l'unité de mélange à une température de 25°C à 60°C et sous une pression de 50 à 90 bars pendant 6 secondes à 5 minutes sous agitation.

2. Procédé selon la revendication 1, **caractérisé en ce que** l'on injecte l'alkylamine sous la forme d'une solution à 10% à 50% en poids et le sucre réducteur sous la forme d'une solution à 40% à 75% en poids dans l'unité de mélange.

3. Procédé selon la revendication 1, **caractérisé en ce que** l'on injecte l'alkylamine sous la forme d'une solution aqueuse à 10% à 50% en poids à une température de 10°C à 30°C et le sucre réducteur sous la forme d'une solution aqueuse à 40% à 75% en poids à une température de 40°C à 60°C dans l'unité de mélange.

4. Procédé selon l'une quelconque ou plusieurs des revendications 1 à 3, **caractérisé en ce que** l'on maintient la solution dans l'unité de mélange à une température de 40°C à 50°C et sous une pression de 60 à 80 bars pendant 6 secondes à 5 minutes sous agitation.

5. Procédé selon l'une quelconque ou plusieurs des revendications 1 à 4, **caractérisé en ce que** l'on maintient la solution dans l'unité de mélange pendant 10 secondes à 1 minute sous agitation.

6. Procédé selon l'une quelconque ou plusieurs des revendications 1 à 5, **caractérisé en ce que** l'on réalise le mélange sous agitation dans un tube résistant à la pression servant d'unité de mélange.

7. Procédé selon l'une quelconque ou plusieurs des revendications 1 à 6, **caractérisé en ce qu'**à la fin du temps de mélange indiqué, on injecte la solution obtenue en vue de son hydrogénation dans un autoclave sous agitation et on l'hydrogène.

8. Procédé selon la revendication 7, **caractérisé en ce que** l'on réalise l'hydrogénation de telle manière que l'on injecte la solution dans un autoclave sous agitation, qui contient du nickel de Raney servant de catalyseur d'hydrogénation à une température de 40°C à 85°C et sous une pression d'hydrogène de 25 à 100 bars, et l'on hydrogène la solution injectée à cette température et sous cette pression d'hydrogène jusqu'à ce que l'hydrogène ne soit quasiment plus absorbé.

9. Procédé selon la revendication 7 ou 8, **caractérisé en ce que** la solution hydrogénée est soumise à une stabilisation thermique en portant la solution, tout en conservant l'hydrogène présent, à une température de 95°C à 110°C en 30 à 150 minutes par élévation continue dé la température et, après avoir atteint cette température, en la refroidissant à une température de 60°C à 90°C.

10. Procédé selon l'une quelconque ou plusieurs des revendications 1 à 9, **caractérisé en ce que** l'on réalise la séparation du produit hydrogéné et soumis à la stabilisation thermique, qui se compose essentiellement de N-alkylpolyhydroxyalkylamine et du catalyseur d'hydrogénation, de telle manière que l'on laisse le catalyseur d'hydrogénation se séparer du produit par sédimentation à une température de 60°C à 90°C et sous une pression d'hydrogène de 70 à 95 bars et que l'on décante la N-alkylpolyhydroxyalkylamine sus-jacente en maintenant cette température et cette pression d'hydrogène.

11. Procédé selon l'une quelconque ou plusieurs des revendications 1 à 10, **caractérisé en ce que** l'on met en oeuvre la monoalkylamine et le sucre réducteur dans un rapport molaire de 1 à 2:1.

12. Procédé selon la revendication 1, **caractérisé en ce que**
a) l'on injecte simultanément la monoalkylamine sous la forme d'une solution aqueuse, alcoolique ou aqueuse/alcoolique à 10% à 50% en poids et le sucre réducteur sous la forme d'une solution aqueuse, alcoolique ou aqueuse/alcoolique à 40% à 95% en poids et dans un rapport molaire de 1 à 1,6 mole d'alkylamine par mole de sucre dans un tube résistant à la pression servant d'unité de mélange, et l'on maintient la solution dans le tube à une température de 25°C à 60°C et sous une pression de 50 à 90 bars pendant 6 secondes à 5 minutes sous agitation,
b) à la fin du temps de mélange indiqué, l'on injecte la solution obtenue à l'étape a) dans un autoclave sous agitation, qui contient 0,1 à 1 mole de monoalkylamine par mole de sucre et de nickel de Raney servant de catalyseur d'hydrogénation mis en oeuvre à une température de 40°C à 85°C et sous une pression d'hydrogène de 50 à 80 bars, et l'on hydrogène la solution injectée à cette température et sous cette pression d'hydrogène, jusqu'à ce que l'hydrogène ne soit quasiment plus absorbé, et l'on soumet la solution hydrogénée à une stabilisation thermique en portant la solution, tout en conservant l'hydrogène présent, à une température de 95°C à 100°C en 60 à 150 minutes par élévation continue de la température et, après avoir atteint cette température, en la refroidissant à une température de 60°C à 90°C, et **en ce que**
c) l'on réalise la séparation du produit hydrogéné et soumis à la stabilisation thermique, qui se compose essentiellement de N-alkylpolyhydroxyalkylamine et du catalyseur d'hydrogénation, de telle manière que l'on laisse le catalyseur d'hydrogénation se séparer du produit par sédimentation à une température de 60°C à 90°C et sous une pression d'hydrogène de 70 à 95 bars, et l'on décante la N-alkylpolyhydroxyalkylamine sus-jacente en maintenant cette température et cette pression d'hydrogène.

13. Procédé selon la revendication 12, **caractérisé en ce que** l'on injecte simultanément la monoalkylamine obtenue à l'étape a) sous la forme d'une solution aqueuse à 10% à 50% en poids à une température de 10°C à 30°C et le sucre réducteur sous la forme d'une solution aqueuse à 40% à 75% en poids à une température de 40°C à 60°C et dans un rapport molaire de 1 à 1,6 mole d'alkylamine par mole de sucre dans le tube, et l'on maintient la solution dans le tube à une température de 40°C à 50°C et sous une pression de 60 à 80 bars pendant 10 secondes à 1 minute sous agitation.
